# EUROPEAN PATENT SPECIFICATION

(11) **EP 3 104 717 B1**
(45) Date of publication and mention of the grant of the patent: **27.05.2020**
(21) Application number: 15706574.9
(22) Date of filing: 04.02.2015
(51) Int. Cl.: A23L 33/17, A23L 33/21, A23L 2/02, A23L 2/58, A23L 2/66, A23L 2/70, A23L 2/84, C12P 19/18, C12N 9/10

(54) **SUCROSE REDUCTION AND GENERATION OF INSOLUBLE FIBER IN JUICES**
SACCHAROSE REDUZIERUNG UND PRODUKTION VON LÖSLICHEN BALASTSTOFFEN IN SÄFTEN
RÉDUCTION DE SACCHAROSE ET LA GÉNÉRATION DE FIBRES INSOLUBLES DANS LES JUS

(30) Priority: 13.02.2014 US 201461939598 P
(43) Date of publication of application: 21.12.2016
(73) Proprietor: Danisco US Inc., Palo Alto, California 94304 (US)
(72) Inventor: GARSKE, Adam L., Palo Alto, California 94304 (US)
(74) Representative: Mewburn Ellis LLP
(86) International application number: PCT/US2015/014403
(87) International publication number: WO 2015/123063

(56) References cited:
- WO-A1-2006/088884
- WO-A1-2008/098975
- WO-A2-00/47727
- WO-A2-2013/036918
- KR-A- 20110 032 480
- US-A1- 2006 127 448
- HANS LEEMHUIS ET AL: "Glucansucrases: Three-dimensional structures, reactions, mechanism, [alpha]-glucan analysis and their implications in biotechnology and food applications", JOURNAL OF BIOTECHNOLOGY, vol. 163, no. 2, 1 January 2013 (2013-01-01), pages 250-272, XP055102852, ISSN: 0168-1656, DOI: 10.1016/j.jbiotec.2012.06.037

## Description

### Cross Reference To Related Application:

This application claims benefit of priority from US provisional application USSN 61/939,598, filed 13 February 2014.

### Field:

The present teachings provide a method of improving the nutritional profile of beverages, especially juices, through enzymatic manipulation to alter sucrose.

### Background:

Studies of human diet increasing show that certain sugars (eg-the disaccharide sucrose) are responsible for a host of human health problems (see for example Lustig, R., Sugar: The Bitter Truth, University of California TV, 2009, 89 minutes). There is a need for improved food and beverages containing less problematic sugars. In addition to removing problematic sugars, methods that produce favorable oligosaccharides (eg-insoluble fiber) are also needed.

U.S. Patent 8,168,242, and U.S. Patent Application 2013/0216652 for descriptions of some aspects of these problems.

The present teachings address these ongoing problems by the use of enzymes.

### Summary:

The present teachings provide a method of making a lower calorie, higher insoluble fiber beverage comprising; treating a sucrose-containing beverage with a glucosyltransferase to convert sucrose to alpha (1-3) glucan to make the lower calorie, higher insoluble fiber beverage according to claim 1.

### Brief description of the drawings:

Fig 1A-1B shows some illustrative data according to the present teachings.
Fig 2 shows some illustrative data according to the present teachings.

### Sequence:

SEQ ID NO: 1

MDETQDKTVTQSNSGTTASLVTSPEATKEADKRTNTKEADVLTPAKETNAVETATTTNTQATAE
AATTATTADVAVAAVPNKEAVVTTDAPAVTTEKAEEQPATVKAEVVNTEVKAPEAALKDSEVEA
ALSLKNIKNIDGKYYYVNEDGSHKENFAITVNGQLLYFGKDGALTSSSTYSFTPGTTNIVDGFS
INNRAYDSSEASFELIDGYLTADSWYRPASIIKDGVTWQASTAEDFRPLLMAWWPNVDTQVNYL
NYMSKVFNLDAKYSSTDKQETLKVAAKDIQIKIEQKIQAEKSTQWLRETISAFVKTQPQWNKET
ENYSKGGGEDHLQGGALLYVNDSRTPWANSDYRRLNRTATNQTGTIDKSILDEQSDPNHMGGFD
FLLANDVDLSNPVVQAEQLNQIHYLMNWGSIVMGDKDANFDGIRVDAVDNVDADMLQLYTNYFR
EYYGVNKSEANALAHISVLEAWSLNDNHYNDKTDGAALAMENKQRLALLFSLAKPIKERTPAVS
PLYNNTFNTTQRDEKTDWINKDGSKAYNEDGTVKQSTIGKYNEKYGDASGNYVFIRAHDNNVQD
IIAEIIKKEINPKSDGFTITDAEMKQAFEIYNKDMLSSDKKYTLNNIPAAYAVMLQNMETITRV
YYGDLYTDDGHYMETKSPYYDTIVNLMKSRIKYVSGGQAQRSYWLPTDGKMDNSDVELYRTNEV
YTSVRYGKDIMTANDTEGSKYSRTSGQVTLVANNPKLNLDQSAKLNVEMGKIHANQKYRALIVG
TADGIKNFTSDADAIAAGYVKETDSNGVLTFGANDIKGYETFDMSGFVAVWVPVGASDNQDIRV
APSTEAKKEGELTLKATEAYDSQLIYEGFSNFQTIPDGSDPSVYTNRKIAENVDLFKSWGVTSF
EMAPQFVSADDGTFLDSVIQNGYAFADRYDLAMSKNNKYGSKEDLRDALKALHKAGIQAIADWV
PDQIYQLPGKEVVTATRTDGAGRKIADAIIDHSLYVANSKSSGKDYQAKYGGEFLAELKAKYPE
MFKVNMISTGKPIDDSVKLKQWKAEYFNGTNVLERGVGYVLSDEATGKYFTVTKEGNFIPLQLT
GKEKVITGFSSDGKGITYFGTSGTQAKSAFVTFNGNTYYFDARGHMVTNSEYSPNGKDVYRFLP
NGIMLSNAFYIDANGNTYLYNSKGQMYKGGYTKFDVSETDKDGKESKVVKFRYFTNEGVMAKGV
TVIDGFTQYFGEDGFQAKDKLVTFKGKTYYFDAHTGNGIKDTWRNINGKWYYFDANGVAATGAQ
VINGQKLYFNEDGSQVKGGVVKNADGTYSKYKEGFGELVTNEFFTTDGNVWYYAGANGKTVTGA
QVINGQHLYFNADGSQVKGGVVKNADGTYSKYNASTGERLTNEFFTTGDNNWYYIGANGKSVTG
EVKIGDDTYFFAKDGKQVKGQTVSAGNGRISYYYGDSGKRAVSTWIEIQPGVYVYFDKNGLAYP
PRVLN

### Detailed description of the inventions:

The practice of the present teachings will employ, unless otherwise indicated, conventional techniques of molecular biology (including recombinant techniques), microbiology, cell biology, biochemistry, and animal feed pelleting, which are within the skill of the art. Such techniques are explained fully in the literature, for example, Molecular Cloning: A Laboratory Manual, second edition (Sambrook et al., 1989); Oligonucleotide Synthesis (M. J. Gait, ed., 1984; Current Protocols in Molecular Biology (F. M. Ausubel et al., eds., 1994); PCR: The Polymerase Chain Reaction (Mullis et al., eds., 1994); Gene Transfer and Expression: A Laboratory Manual (Kriegler, 1990), and The Alcohol Textbook (Ingledew et al., eds., Fifth Edition, 2009), and Essentials of Carbohydrate Chemistry and Biochemistry (Lindhorste, 2007).

Unless defined otherwise herein, all technical and scientific terms used herein have the same meaning as commonly understood by one of ordinary skill in the art to which the present teachings belong. Singleton, et al., Dictionary of Microbiology and Molecular Biology, second ed., John Wiley and Sons, New York (1994), and Hale & Markham, The Harper Collins Dictionary of Biology, Harper Perennial, NY (1991) provide one of skill with a general dictionary of many of the terms used in this invention. Any methods and materials similar or equivalent to those described herein can be used in the practice or testing of the present teachings.

Numeric ranges provided herein are inclusive of the numbers defining the range.

### Definitions:

As used herein, "immobilized" refers to any of a variety of approaches of using supports to reduce the movement of the glucosyltransferase enzyme, including covalent binding, entrapment, physical adsorption, and cross-linking. Illustrative immobilization approaches are found in EP 1379674B1, EP0641859 and references cited therein.

As used herein, "sucrose-containing beverage" refers to any drink containing a sufficient amount of sucrose to benefit for the sucrose-reducing approaches of the present teachings, and includes juices (eg apple and orange).

As used herein, "alpha (1-3) glucan" refers to an oligosaccharide containing alpha 1-3 bonds between glucose monomers.

As used herein, "glucosyltransferase from Streptococcus salivarius" refers to an enzyme as generally taught by illustration in Giffard et al., J. Gen Microbiol. 1993 Jul; 139(7): 1511-22, and Simpson et al., Microbiology (1995), 141, 1451-1460. It is also referred to herein as "GtfJ". An exemplified enzyme is provided herein as SEQ ID NO: 1.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide.

Reference to the wild-type polypeptide is understood to include the mature form of the polypeptide. A "mature" polypeptide or variant, thereof, is one in which a signal sequence is absent, for example, cleaved from an immature form of the polypeptide during or following expression of the polypeptide.

The term "variant," with respect to a polypeptide, refers to a polypeptide that differs from a specified wild-type, parental, or reference polypeptide in that it includes one or more naturally-occurring or man-made substitutions, insertions, or deletions of an amino acid. Similarly, the term "variant," with respect to a polynucleotide, refers to a polynucleotide that differs in nucleotide sequence from a specified wild-type, parental, or reference polynucleotide. The identity of the wild-type, parental, or reference polypeptide or polynucleotide will be apparent from context.

The term "recombinant," when used in reference to a subject cell, nucleic acid, protein or vector, indicates that the subject has been modified from its native state. Thus, for example, recombinant cells express genes that are not found within the native (non-recombinant) form of the cell, or express native genes at different levels or under different conditions than found in nature. Recombinant nucleic acids differ from a native sequence by one or more nucleotides and/or are operably linked to heterologous sequences, *e.g*., a heterologous promoter in an expression vector. Recombinant proteins may differ from a native sequence by one or more amino acids and/or are fused with heterologous sequences. A vector comprising a nucleic acid encoding a glucosyltransferase is a recombinant vector.

The terms "recovered," "isolated," and "separated," refer to a compound, protein (polypeptides), cell, nucleic acid, amino acid, or other specified material or component that is removed from at least one other material or component with which it is naturally associated as found in nature. An "isolated" polypeptides, thereof, includes, but is not limited to, a culture broth containing secreted polypeptide expressed in a heterologous host cell.

The term "purified" refers to material (*e.g*., an isolated polypeptide or polynucleotide) that is in a relatively pure state, *e.g*., at least about 90% pure, at least about 95% pure, at least about 98% pure, or even at least about 99% pure.

The term "enriched" refers to material (*e.g*., an isolated polypeptide or polynucleotide) that is in about 50% pure, at least about 60% pure, at least about 70% pure, or even at least about 70% pure.

A "pH range," with reference to an enzyme, refers to the range of pH values under which the enzyme exhibits catalytic activity.

The terms "pH stable" and "pH stability," with reference to an enzyme, relate to the ability of the enzyme to retain activity over a wide range of pH values for a predetermined period of time (*e.g*., 15 min., 30 min., 1 hour).

The term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e.,* N→C).

The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

"Hybridization" refers to the process by which one strand of nucleic acid forms a duplex with, *i.e.,* base pairs with, a complementary strand, as occurs during blot hybridization techniques and PCR techniques. Stringent hybridization conditions are exemplified by hybridization under the following conditions: 65°C and 0.1X SSC (where 1X SSC = 0.15 M NaCl, 0.015 M Na₃ citrate, pH 7.0). Hybridized, duplex nucleic acids are characterized by a melting temperature (Tₘ), where one half of the hybridized nucleic acids are unpaired with the complementary strand. Mismatched nucleotides within the duplex lower the Tₘ. Very stringent hybridization conditions involve 68°C and 0.1X SSC

A "synthetic" molecule is produced by *in vitro* chemical or enzymatic synthesis rather than by an organism.

The terms "transformed," "stably transformed," and "transgenic," used with reference to a cell means that the cell contains a non-native (*e.g*., heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest (*e.g*., an glucosyltransferase) has been introduced. Exemplary host strains are microorganism cells (*e.g*., bacteria, filamentous fungi, and yeast) capable of expressing the polypeptide of interest. The term "host cell" includes protoplasts created from cells.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

The term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (*e.g*., hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequences.

A "signal sequence" is a sequence of amino acids attached to the N-terminal portion of a protein, which facilitates the secretion of the protein outside the cell. The mature form of an extracellular protein lacks the signal sequence, which is cleaved off during the secretion process.

"Biologically active" refers to a sequence having a specified biological activity, such an enzymatic activity.

The term "specific activity" refers to the number of moles of substrate that can be converted to product by an enzyme or enzyme preparation per unit time under specific conditions. Specific activity is generally expressed as units (U)/mg of protein.

As used herein, "percent sequence identity" means that a particular sequence has at least a certain percentage of amino acid residues identical to those in a specified reference sequence, when aligned using the CLUSTAL W algorithm with default parameters. *See* Thompson et al. (1994) Nucleic Acids Res. 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

Deletions are counted as non-identical residues, compared to a reference sequence. Deletions occurring at either termini are included. For example, a variant with five amino acid deletions of the C-terminus of the mature 617 residue polypeptide would have a percent sequence identity of 99% (612 / 617 identical residues × 100, rounded to the nearest whole number) relative to the mature polypeptide. Such a variant would be encompassed by a variant having "at least 99% sequence identity" to a mature polypeptide.

"Fused" polypeptide sequences are connected, *i.e.,* operably linked, via a peptide bond between two subject polypeptide sequences.

The term "filamentous fungi" refers to all filamentous forms of the subdivision Eumycotina, particularly Pezizomycotina species.

The term "about" refers to ± 5% to the referenced value.

### Additional mutations

In some embodiments, the present glucosyltransferases further include one or more mutations that provide a further performance or stability benefit. Exemplary performance benfits include but are not limited to increased thermal stability, increased storage stability, increased solubility, an altered pH profile, decreased calcium dependence, increased specific activity, modified substrate specificity, modified substrate binding, modified pH-dependent activity, modified pH-dependent stability, increased oxidative stability, and increased expression. In some cases, the performance benefit is realized at a relatively low temperature. In some cases, the performance benefit is realized at relatively high temperature.

Furthermore, the present glucosyltransferases may include any number of conservative amino acid substitutions. Exemplary conservative amino acid substitutions are listed in the following Table.

### Conservative amino acid substitutions

| *For Amino Acid* | *Code* | *Replace with any of* |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

The reader will appreciate that some of the above mentioned conservative mutations can be produced by genetic manipulation, while others are produced by introducing synthetic amino acids into a polypeptide by genetic or other means.

The present glucosyltransferase may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective glucosyltransferase polypeptides. The present glucosyltransferase polypeptides may also be truncated to remove the N or C-termini, so long as the resulting polypeptides retain glucosyltransferase activity.

The present glucosyltransferase may be a "chimeric" or "hybrid" polypeptide, in that it includes at least a portion of a first glucosyltransferase polypeptide, and at least a portion of a second glucosyltransferase polypeptide. The present glucosyltransferase may further include heterologous signal sequence, an epitope to allow tracking or purification, or the like. Exemplary heterologous signal sequences are from *B. licheniformis* amylase (LAT), B. *subtilis* (AmyE or AprE), and *Streptomyces* CeIA.

### Production of Variant glucosyltransferases

The present glucosyltransferase can be produced in host cells, for example, by secretion or intracellular expression. A cultured cell material (*e.g*., a whole-cell broth) comprising a glucosyltransferase can be obtained following secretion of the glucosyltransferase into the cell medium. Optionally, the glucosyltransferase can be isolated from the host cells, or even isolated from the cell broth, depending on the desired purity of the final glucosyltransferase. A gene encoding a glucosyltransferase can be cloned and expressed according to methods well known in the art. Suitable host cells include bacterial, fungal (including yeast and filamentous fungi), and plant cells (including algae). Particularly useful host cells include *Aspergillus niger, Aspergillus oryzae* or *Trichoderma reesei.* Other host cells include bacterial cells, *e.g., Bacillus subtilis* or *B. licheniformis,* as well as *Streptomyces, E. Coli.*

The host cell further may express a nucleic acid encoding a homologous or heterologous glucosyltransferase, *i.e.,* a glucosyltransferase that is not the same species as the host cell, or one or more other enzymes. The glucosyltransferase may be a variant glucosyltransferase. Additionally, the host may express one or more accessory enzymes, proteins, peptides.

### Vectors

A DNA construct comprising a nucleic acid encoding a glucosyltransferase can be constructed to be expressed in a host cell. Because of the well-known degeneracy in the genetic code, variant polynucleotides that encode an identical amino acid sequence can be designed and made with routine skill. It is also well-known in the art to optimize codon use for a particular host cell. Nucleic acids encoding glucosyltransferase can be incorporated into a vector. Vectors can be transferred to a host cell using well-known transformation techniques, such as those disclosed below.

The vector may be any vector that can be transformed into and replicated within a host cell. For example, a vector comprising a nucleic acid encoding a glucosyltransferase can be transformed and replicated in a bacterial host cell as a means of propagating and amplifying the vector. The vector also may be transformed into an expression host, so that the encoding nucleic acids can be expressed as a functional glucosyltransferase. Host cells that serve as expression hosts can include filamentous fungi, for example. The Fungal Genetics Stock Center (FGSC) Catalogue of Strains lists suitable vectors for expression in fungal host cells. *See* FGSC, Catalogue of Strains, University of Missouri, *at* www.fgsc.net (last modified January 17, 2007). A representative vector is pJG153, a promoterless Cre expression vector that can be replicated in a bacterial host. *See* Harrison et al. (June 2011) Applied Environ. Microbiol. 77: 3916-22. pJG153can be modified with routine skill to comprise and express a nucleic acid encoding a glucosyltransferase.

A nucleic acid encoding a glucosyltransferase can be operably linked to a suitable promoter, which allows transcription in the host cell. The promoter may be any DNA sequence that shows transcriptional activity in the host cell of choice and may be derived from genes encoding proteins either homologous or heterologous to the host cell. Exemplary promoters for directing the transcription of the DNA sequence encoding a glucosyltransferase, especially in a bacterial host, are the promoter of the lac operon of *E. coli,* the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus niger* neutral α-amylase, A. *niger* acid stable α-amylase, *A. niger* glucoamylase, *Rhizomucor miehei* lipase, *A. oryzae* alkaline protease, *A. oryzae* triose phosphate isomerase, or *A. nidulans* acetamidase. When a gene encoding a glucosyltransferase is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. *cbh1* is an endogenous, inducible promoter from *T. reesei. See* Liu et al. (2008) "Improved heterologous gene expression in Trichoderma reesei by cellobiohydrolase I gene (cbh1) promoter optimization," Acta Biochim. Biophys. Sin (Shanghai) 40(2): 158-65.

The coding sequence can be operably linked to a signal sequence. The DNA encoding the signal sequence may be the DNA sequence naturally associated with the glucosyltransferase gene to be expressed or from a different Genus or species. A signal sequence and a promoter sequence comprising a DNA construct or vector can be introduced into a fungal host cell and can be derived from the same source. For example, the signal sequence is the *cbh1* signal sequence that is operably linked to a *cbh1* promoter.

An expression vector may also comprise a suitable transcription terminator and, in eukaryotes, polyadenylation sequences operably linked to the DNA sequence encoding a variant glucosyltransferase. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUB110, pE194, pAMB1, and pIJ702.

The vector may also comprise a selectable marker, *e.g*., a gene the product of which complements a defect in the isolated host cell, such as the *dal* genes from *B. subtilis* or *B. licheniformis,* or a gene that confers antibiotic resistance such as, *e.g.,* ampicillin, kanamycin, chloramphenicol or tetracycline resistance. Furthermore, the vector may comprise *Aspergillus* selection markers such as *amdS, argB, niaD* and *xxsC,* a marker giving rise to hygromycin resistance, or the selection may be accomplished by co-transformation, such as known in the art. *See e.g.,* International PCT Application WO 91/17243.

Intracellular expression may be advantageous in some respects, *e.g*., when using certain bacteria or fungi as host cells to produce large amounts of glucosyltransferase for subsequent enrichment or purification. Extracellular secretion of glucosyltransferase into the culture medium can also be used to make a cultured cell material comprising the isolated glucosyltransferase.

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the glucosyltransferase to a host cell organelle such as a peroxisome, or to a particular host cell compartment. Such a targeting sequence includes but is not limited to the sequence, SKL. For expression under the direction of control sequences, the nucleic acid sequence of the glucosyltransferase is operably linked to the control sequences in proper manner with respect to expression.

The procedures used to ligate the DNA construct encoding a glucosyltransferase, the promoter, terminator and other elements, respectively, and to insert them into suitable vectors containing the information necessary for replication, are well known to persons skilled in the art (*see, e.g.,* Sambrook et al., MOLECULAR CLONING: A LABORATORY MANUAL, 2nd ed., Cold Spring Harbor, 1989, and 3rd ed., 2001).

### Transformation and Culture of Host Cells

An isolated cell, either comprising a DNA construct or an expression vector, is advantageously used as a host cell in the recombinant production of a glucosyltransferase. The cell may be transformed with the DNA construct encoding the enzyme, conveniently by integrating the DNA construct (in one or more copies) in the host chromosome. This integration is generally considered to be an advantage, as the DNA sequence is more likely to be stably maintained in the cell. Integration of the DNA constructs into the host chromosome may be performed according to conventional methods, *e.g*., by homologous or heterologous recombination. Alternatively, the cell may be transformed with an expression vector as described above in connection with the different types of host cells.

Examples of suitable bacterial host organisms are Gram positive bacterial species such as *Bacillaceae* including *Bacillus subtilis, Bacillus licheniformis, Bacillus lentus, Bacillus brevis, Geobacillus* (formerly *Bacillus*) *stearothermophilus, Bacillus alkalophilus, Bacillus amyloliquefaciens, Bacillus coagulans, Bacillus lautus, Bacillus megaterium,* and *Bacillus thuringiensis; Streptomyces* species such as *Streptomyces murinus;* lactic acid bacterial species including *Lactococcus* sp. such as *Lactococcus lactis; Lactobacillus* sp. including *Lactobacillus reuteri; Leuconostoc* sp.; *Pediococcus* sp.; and *Streptococcus* sp. Alternatively, strains of a Gram negative bacterial species belonging to *Enterobacteriaceae* including *E. coli,* or to *Pseudomonadaceae* can be selected as the host organism.

A suitable yeast host organism can be selected from the biotechnologically relevant yeasts species such as but not limited to yeast species such as *Pichia* sp., *Hansenula* sp., or *Kluyveromyces, Yarrowinia, Schizosaccharomyces* species or a species of *Saccharomyces,* including *Saccharomyces cerevisiae* or a species belonging to *Schizosaccharomyces* such as, for example, S. *pombe* species. A strain of the methylotrophic yeast species, *Pichia pastoris,* can be used as the host organism. Alternatively, the host organism can be a *Hansenula* species. Suitable host organisms among filamentous fungi include species of *Aspergillus, e.g., Aspergillus niger, Aspergillus oryzae, Aspergillus tubigensis, Aspergillus awamori,* or *Aspergillus nidulans.* Alternatively, strains of a *Fusarium* species, *e.g., Fusarium oxysporum* or of a *Rhizomucor* species such as *Rhizomucor miehei* can be used as the host organism. Other suitable strains include *Thermomyces* and *Mucor* species. In addition, *Trichoderma sp.* can be used as a host. A suitable procedure for transformation of *Aspergillus* host cells includes, for example, that described in EP 238023. A glucosyltransferase expressed by a fungal host cell can be glycosylated, *i.e.,* will comprise a glycosyl moiety. The glycosylation pattern can be the same or different as present in the wild-type glucosyltransferase. The type and/or degree of glycosylation may impart changes in enzymatic and/or biochemical properties.

It is advantageous to delete genes from expression hosts, where the gene deficiency can be cured by the transformed expression vector. Known methods may be used to obtain a fungal host cell having one or more inactivated genes. Gene inactivation may be accomplished by complete or partial deletion, by insertional inactivation or by any other means that renders a gene nonfunctional for its intended purpose, such that the gene is prevented from expression of a functional protein. Any gene from a *Trichoderma* sp. or other filamentous fungal host that has been cloned can be deleted, for example, *cbh1, cbh2, egl1,* and *egl2* genes. Gene deletion may be accomplished by inserting a form of the desired gene to be inactivated into a plasmid by methods known in the art.

Introduction of a DNA construct or vector into a host cell includes techniques such as transformation; electroporation; nuclear microinjection; transduction; transfection, *e.g*., lipofection mediated and DEAE-Dextrin mediated transfection; incubation with calcium phosphate DNA precipitate; high velocity bombardment with DNA-coated microprojectiles; and protoplast fusion. General transformation techniques are known in the art. *See, e.g.,* Sambrook *et al.* (2001), *supra.* The expression of heterologous protein in *Trichoderma* is described, for example, in U.S. Patent No. 6,022,725. Reference is also made to Cao et al. (2000) Science 9:991-1001 for transformation of *Aspergillus* strains. Genetically stable transformants can be constructed with vector systems whereby the nucleic acid encoding a glucosyltransferase is stably integrated into a host cell chromosome. Transformants are then selected and purified by known techniques.

The preparation of *Trichoderma* sp. for transformation, for example, may involve the preparation of protoplasts from fungal mycelia. *See* Campbell et al. (1989) Curr. Genet. 16: 53-56. The mycelia can be obtained from germinated vegetative spores. The mycelia are treated with an enzyme that digests the cell wall, resulting in protoplasts. The protoplasts are protected by the presence of an osmotic stabilizer in the suspending medium. These stabilizers include sorbitol, mannitol, potassium chloride, magnesium sulfate, and the like. Usually the concentration of these stabilizers varies between 0.8 M and 1.2 M, *e.g.,* a 1.2 M solution of sorbitol can be used in the suspension medium.

Uptake of DNA into the host *Trichoderma* sp. strain depends upon the calcium ion concentration. Generally, between about 10-50 mM CaCl₂ is used in an uptake solution. Additional suitable compounds include a buffering system, such as TE buffer (10 mM Tris, pH 7.4; 1 mM EDTA) or 10 mM MOPS, pH 6.0 and polyethylene glycol. The polyethylene glycol is believed to fuse the cell membranes, thus permitting the contents of the medium to be delivered into the cytoplasm of the *Trichoderma* sp. strain. This fusion frequently leaves multiple copies of the plasmid DNA integrated into the host chromosome.

Usually transformation of *Trichoderma* sp. uses protoplasts or cells that have been subjected to a permeability treatment, typically at a density of 10⁵ to 10⁷/mL, particularly 2x10⁶/mL. A volume of 100 µL of these protoplasts or cells in an appropriate solution (e.g., 1.2 M sorbitol and 50 mM CaCl₂) may be mixed with the desired DNA. Generally, a high concentration of PEG is added to the uptake solution. From 0.1 to 1 volume of 25% PEG 4000 can be added to the protoplast suspension; however, it is useful to add about 0.25 volumes to the protoplast suspension. Additives, such as dimethyl sulfoxide, heparin, spermidine, potassium chloride and the like, may also be added to the uptake solution to facilitate transformation. Similar procedures are available for other fungal host cells. *See, e.g.,* U.S. Patent No. 6,022,725.

### Expression

A method of producing a glucosyltransferase may comprise cultivating a host cell as described above under conditions conducive to the production of the enzyme and recovering the enzyme from the cells and/or culture medium.

The medium used to cultivate the cells may be any conventional medium suitable for growing the host cell in question and obtaining expression of a glucosyltransferase. Suitable media and media components are available from commercial suppliers or may be prepared according to published recipes (*e.g*., as described in catalogues of the American Type Culture Collection).

An enzyme secreted from the host cells can be used in a whole broth preparation. In the present methods, the preparation of a spent whole fermentation broth of a recombinant microorganism can be achieved using any cultivation method known in the art resulting in the expression of a glucosyltransferase. Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the glucosyltransferase to be expressed or isolated. The term "spent whole fermentation broth" is defined herein as unfractionated contents of fermentation material that includes culture medium, extracellular proteins (*e.g*., enzymes), and cellular biomass. It is understood that the term "spent whole fermentation broth" also encompasses cellular biomass that has been lysed or permeabilized using methods well known in the art.

An enzyme secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

The polynucleotide encoding a glucosyltransferase in a vector can be operably linked to a control sequence that is capable of providing for the expression of the coding sequence by the host cell, *i.e.* the vector is an expression vector. The control sequences may be modified, for example by the addition of further transcriptional regulatory elements to make the level of transcription directed by the control sequences more responsive to transcriptional modulators. The control sequences may in particular comprise promoters.

Host cells may be cultured under suitable conditions that allow expression of a glucosyltransferase. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or Sophorose. Polypeptides can also be produced recombinantly in an *in vitro* cell-free system, such as the TNT™ (Promega) rabbit reticulocyte system.

An expression host also can be cultured in the appropriate medium for the host, under aerobic conditions. Shaking or a combination of agitation and aeration can be provided, with production occurring at the appropriate temperature for that host, *e.g*., from about 25°C to about 75°C (*e.g*., 30°C to 45°C), depending on the needs of the host and production of the desired glucosyltransferase. Culturing can occur from about 12 to about 100 hours or greater (and any hour value there between, *e.g*., from 24 to 72 hours). Typically, the culture broth is at a pH of about 4.0 to about 8.0, again depending on the culture conditions needed for the host relative to production of a glucosyltransferase.

### Methods for Enriching and Purifying glucosyltransferases

Fermentation, separation, and concentration techniques are well known in the art and conventional methods can be used in order to prepare a glucosyltransferase polypeptide-containing solution.

After fermentation, a fermentation broth is obtained, the microbial cells and various suspended solids, including residual raw fermentation materials, are removed by conventional separation techniques in order to obtain a glucosyltransferase solution. Filtration, centrifugation, microfiltration, rotary vacuum drum filtration, ultrafiltration, centrifugation followed by ultra-filtration, extraction, or chromatography, or the like, are generally used.

It is desirable to concentrate a glucosyltransferase polypeptide-containing solution in order to optimize recovery. Use of unconcentrated solutions requires increased incubation time in order to collect the enriched or purified enzyme precipitate.

The enzyme containing solution is concentrated using conventional concentration techniques until the desired enzyme level is obtained. Concentration of the enzyme containing solution may be achieved by any of the techniques discussed herein. Exemplary methods of enrichment and purification include but are not limited to rotary vacuum filtration and/or ultrafiltration.

The enzyme solution is concentrated into a concentrated enzyme solution until the enzyme activity of the concentrated glucosyltransferase polypeptide-containing solution is at a desired level.

Concentration may be performed using, *e.g*., a precipitation agent, such as a metal halide precipitation agent. Metal halide precipitation agents include but are not limited to alkali metal chlorides, alkali metal bromides and blends of two or more of these metal halides. Exemplary metal halides include sodium chloride, potassium chloride, sodium bromide, potassium bromide and blends of two or more of these metal halides. The metal halide precipitation agent, sodium chloride, can also be used as a preservative.

The metal halide precipitation agent is used in an amount effective to precipitate a glucosyltransferase. The selection of at least an effective amount and an optimum amount of metal halide effective to cause precipitation of the enzyme, as well as the conditions of the precipitation for maximum recovery including incubation time, pH, temperature and concentration of enzyme, will be readily apparent to one of ordinary skill in the art, after routine testing.

Generally, at least about 5% w/v (weight/volume) to about 25% w/v of metal halide is added to the concentrated enzyme solution, and usually at least 8% w/v. Generally, no more than about 25% w/v of metal halide is added to the concentrated enzyme solution and usually no more than about 20% w/v. The optimal concentration of the metal halide precipitation agent will depend, among others, on the nature of the specific glucosyltransferase polypeptide and on its concentration in the concentrated enzyme solution.

Another alternative way to precipitate the enzyme is to use organic compounds. Exemplary organic compound precipitating agents include: 4-hydroxybenzoic acid, alkali metal salts of 4-hydroxybenzoic acid, alkyl esters of 4-hydroxybenzoic acid, and blends of two or more of these organic compounds. The addition of the organic compound precipitation agents can take place prior to, simultaneously with or subsequent to the addition of the metal halide precipitation agent, and the addition of both precipitation agents, organic compound and metal halide, may be carried out sequentially or simultaneously.

Generally, the organic precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitation agents can be, for example, linear or branched alkyl esters of 4-hydroxybenzoic acid, wherein the alkyl group contains from 1 to 10 carbon atoms, and blends of two or more of these organic compounds. Exemplary organic compounds are linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl esters of 4-hydroxybenzoic acid, butyl ester of 4-hydroxybenzoic acid, ethyl ester of 4-hydroxybenzoic acid and blends of two or more of these organic compounds can also be used. Additional organic compounds also include but are not limited to 4-hydroxybenzoic acid methyl ester (named methyl PARABEN), 4-hydroxybenzoic acid propyl ester (named propyl PARABEN), which also are both preservative agents. For further descriptions, *see, e.g.,* U.S. Patent No. 5,281,526.

Addition of the organic compound precipitation agent provides the advantage of high flexibility of the precipitation conditions with respect to pH, temperature, glucosyltransferase concentration, precipitation agent concentration, and time of incubation.

The organic compound precipitation agent is used in an amount effective to improve precipitation of the enzyme by means of the metal halide precipitation agent. The selection of at least an effective amount and an optimum amount of organic compound precipitation agent, as well as the conditions of the precipitation for maximum recovery including incubation time, pH, temperature and concentration of enzyme, will be readily apparent to one of ordinary skill in the art, in light of the present disclosure, after routine testing.

Generally, at least about 0.01% w/v of organic compound precipitation agent is added to the concentrated enzyme solution and usually at least about 0.02% w/v. Generally, no more than about 0.3% w/v of organic compound precipitation agent is added to the concentrated enzyme solution and usually no more than about 0.2% w/v.

The concentrated polypeptide solution, containing the metal halide precipitation agent, and the organic compound precipitation agent, can be adjusted to a pH, which will, of necessity, depend on the enzyme to be enriched or purified. Generally, the pH is adjusted at a level near the isoelectric point of the glucosyltransferase. The pH can be adjusted at a pH in a range from about 2.5 pH units below the isoelectric point (pl) up to about 2.5 pH units above the isoelectric point.

The incubation time necessary to obtain an enriched or purified enzyme precipitate depends on the nature of the specific enzyme, the concentration of enzyme, and the specific precipitation agent(s) and its (their) concentration. Generally, the time effective to precipitate the enzyme is between about 1 to about 30 hours; usually it does not exceed about 25 hours. In the presence of the organic compound precipitation agent, the time of incubation can still be reduced to less about 10 hours and in most cases even about 6 hours.

Generally, the temperature during incubation is between about 4°C and about 50°C. Usually, the method is carried out at a temperature between about 10°C and about 45°C (*e.g*., between about 20°C and about 40°C). The optimal temperature for inducing precipitation varies according to the solution conditions and the enzyme or precipitation agent(s) used.

The overall recovery of enriched or purified enzyme precipitate, and the efficiency with which the process is conducted, is improved by agitating the solution comprising the enzyme, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the enriched or purified enzyme is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration, or the like. Further enrichment or purification of the enzyme precipitate can be obtained by washing the precipitate with water. For example, the enriched or purified enzyme precipitate is washed with water containing the metal halide precipitation agent, or with water containing the metal halide and the organic compound precipitation agents.

During fermentation, a glucosyltransferase polypeptide accumulates in the culture broth. For the isolation, enrichment, or purification of the desired glucosyltransferase, the culture broth is centrifuged or filtered to eliminate cells, and the resulting cell-free liquid is used for enzyme enrichment or purification. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme-active fraction. For further enrichment or purification, a conventional procedure such as ion exchange chromatography may be used.

Enriched or purified enzymes can be made into a final product that is either liquid (solution, slurry) or solid (granular, powder).

A more specific example of enrichment or purification, is described in Sumitani et al. (2000) "New type of starch-binding domain: the direct repeat motif in the C-terminal region of Bacillus sp. 195 α-amylase contributes to starch binding and raw starch degrading," Biochem. J. 350: 477-484, and is briefly summarized here. The enzyme obtained from 4 liters of a *Streptomyces lividans* TK24 culture supernatant is treated with (NH₄)₂SO₄ at 80% saturation. The precipitate is recovered by centrifugation at 10,000 × g (20 min. and 4°C) and re-dissolved in 20 mM Tris/HCI buffer (pH 7.0) containing 5 mM CaCl₂. The solubilized precipitate is then dialyzed against the same buffer. The dialyzed sample is then applied to a Sephacryl S-200 column, which had previously been equilibrated with 20 mM Tris/HCI buffer, (pH 7.0), 5 mM CaCl₂, and eluted at a linear flow rate of 7 mL/hr with the same buffer. Fractions from the column are collected and assessed for activity as judged by enzyme assay and SDS-PAGE. The protein is further purified as follows. A Toyopearl HW55 column (Tosoh Bioscience, Montgomeryville, PA; Cat. No. 19812) is equilibrated with 20 mM Tris/HCI buffer (pH 7.0) containing 5 mM CaCl₂ and 1.5 M (NH₄)₂SO₄. The enzyme is eluted with a linear gradient of 1.5 to 0 M (NH₄)₂SO₄ in 20 mM Tris/HCL buffer, pH 7.0 containing 5 mM CaCl₂. The active fractions are collected, and the enzyme precipitated with (NH₄)₂SO₄ at 80% saturation. The precipitate is recovered, re-dissolved, and dialyzed as described above. The dialyzed sample is then applied to a Mono Q HR5/5 column (Amersham Pharmacia; Cat. No. 17-5167-01) previously equilibrated with 20 mM Tris/HCI buffer (pH 7.0) containing 5 mM CaCl₂, at a flow rate of 60 mL/hour. The active fractions are collected and added to a 1.5 M (NH₄)₂SO₄ solution. The active enzyme fractions are re-chromatographed on a Toyopearl HW55 column, as before, to yield a homogeneous enzyme as determined by SDS-PAGE. *See* Sumitani et al. (2000) Biochem. J. 350: 477-484, for general discussion of the method and variations thereon.

For production scale recovery, glucosyltransferase polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules.

The present teachings provide a strategy for generating insoluble fibers in fruit juices using a glucosyltransferase. The glucosyltransferase uses a sucrose substrate and produces alpha (1-3) glucan polymers, in addition to leucrose, fructose and short oligosaccharides. Since the resulting alpha (1-3) glucans are not digested by human enzymes, the resulting beverage is improved.

The glucosyltransferase is that from Streptococcus salivarius (SEQ ID NO: 1), or anything 80%, 85%, 90%, 95%, 98%, 99%, or 99.9% identical thereto. In some embodiments, the enzyme can be immobilized, which can facilitate repeated use. For example, the enzyme can be immobilized on a solid support, and liquids of interest (eg juices) flowed across the solid support, thus effectuating the conversion of sucrose to alpha (1-3) glucan polymers. These alpha (1-3) glucan polymers can be retained, thereby allowing the juice flow-through to be clarified. Alternatively, the alpha (1-3) polymers can be released into the flow-through juice, thus providing insoluble fiber in the resulting juice.

### Example:

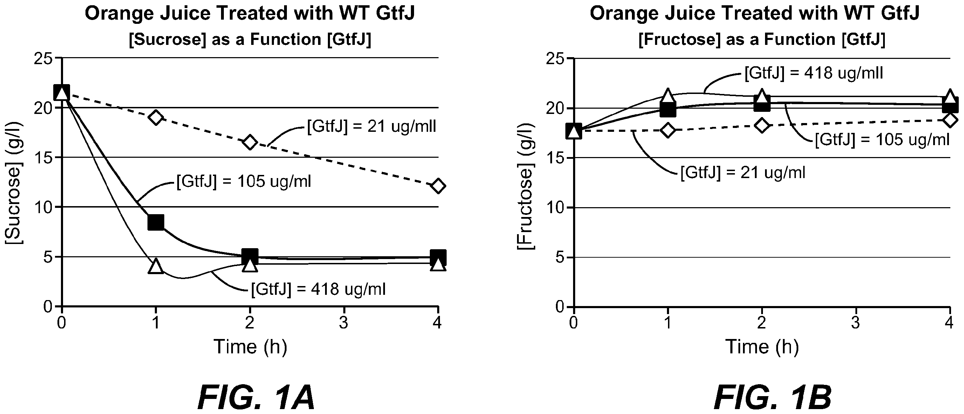

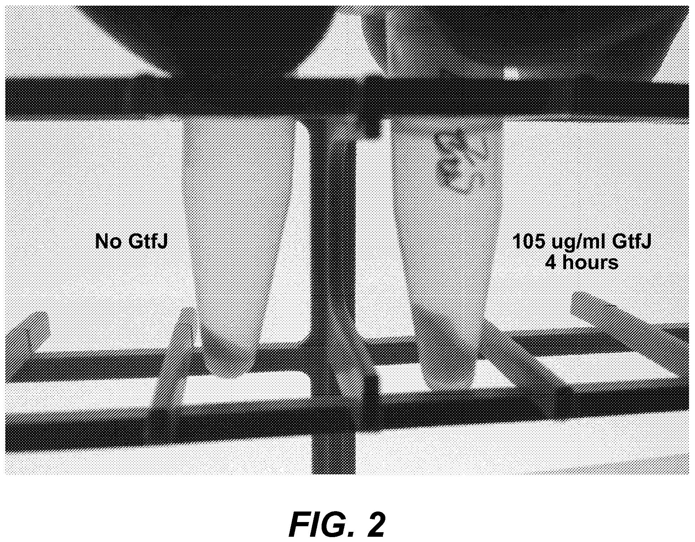

SEQ ID NO: 1 was expressed and purified from E. Coli using conventional methods (see for example Microbiology (1995), 141, 1451-1460), and explored in the experiments depicted in Figures 1A-1B and 2. In Figure 1A-1B, orange juice was treated with wild type GTFJ. The reagent Simply Orange TM was diluted 1:1 with NaOAc, pH 5.5 and assayed. Sucrose level was reduced by 80% at equilibrium. There was about a 30% loss in sweetness, though this could be an overestimate due to other sugars being formed (e.g. leucrose). Figure 2 shows the production of substantial insoluble fiber, generating about 4.4 g alpha-glucan per liter of OJ. This has the effect of decreasing glycemic index, reduces calories, and likely involves little viscosity change. The No GTFJ sample is shown in the left tube, and the 105 ug/ml GTFJ 4 hours tube is on the right.

## Claims

1. A method of making a lower calorie, higher insoluble fiber beverage comprising; treating a sucrose-containing beverage with a glucosyltransferase to convert sucrose to alpha (1-3) glucan to make the lower calorie, higher insoluble fiber beverage;
wherein the treating comprises treating with an enzyme having at least 80% sequence identity to the glucosyltransferase having the amino acid sequence set out in SEQ ID NO:1 and wherein the beverage comprises a sucrose level reduced at least 30%.

2. The method of claim 1, further comprising removing the alpha (1-3) glucan to make a lower calorie, clarified beverage.

3. The method of claim 1 or claim 2, wherein the lower calorie, higher insoluble fiber beverage contains no less than 95%, 96%, 97%, 98%, 99%, or 99.9% fructose of the sucrose-containing beverage.

4. The method according to any of the preceding claims, wherein the treating comprises treating with a glucosyltransferase comprising SEQ. ID NO: 1.

5. The method according to any of the preceding claims, wherein the treating comprises treating with an enzyme having at least 85% 90%, 95%, 98%, 99%, or 99. 9% sequence identity to the glucosyltransferease having the amino acid sequence set out in SEQ ID NO:1.

6. The method according to any of the preceding claims wherein the treating comprises treating with an immobilized glucosyltransferase.

7. The method according to any of the preceding claims wherein the treating comprises treating with an immobilized glucosyltransferase from *Streptococcus salivarius.*

8. The method according to any of the preceding claims, wherein the beverage comprises a fruit juice.

9. The method according to any of the preceding claims wherein the beverage comprises apple juice or orange juice.

10. The method according to any of the preceding claims wherein the beverage comprises a sucrose level reduced, at least 40%, at least 50%, at least 60%, at least 70%, at least 80%, at least 85%, or at least 90% compared to a control beverage lacking the treating.

11. The method of any one of the preceding claims, wherein the beverage comprises at least 2, at least 2.5, at least 3, at least 4, or at least 4.3 grams of alpha (1-3) glucans per liter.

12. The method of claim 11, wherein the beverage is orange juice or apple juice, and wherein it contains no less than 95%, 96%, 97%, 98%, 99%, or 99.9% fructose of average orange juice or apple juice.

## Patentansprüche

1. Verfahren zur Herstellung eines kaloriereduzierten Getränks höheren unlöslichen Ballaststoffgehalts, umfassend: Behandlung eines sucrosehaltigen Getränks mit einer Glucosyltransferase, um Sucrose in Alpha-(1-3)-glucan umzuwandeln, um das kaloriereduzierte Getränk höheren unlöslichen Ballaststoffgehalts herzustellen;
wobei das Behandeln das Behandeln mit einem Enzym umfasst, das mindestens 80% Sequenzidentität mit der Glucosyltransferase aufweist, die die in SEQ ID NO:1 aufgeführte Aminosäuresequenz aufweist, und wobei das Getränk ein um mindestens 30% reduziertes Sucroseniveau umfasst.

2. Verfahren nach Anspruch 1, das ferner das Entfernen des Alpha-(1-3)-glucans zum Herstellen eines kaloriereduzierten geklärten Getränks umfasst.

3. Verfahren nach Anspruch 1 oder Anspruch 2, wobei das kaloriereduzierte Getränk höheren unlöslichen Ballaststoffgehalts, nicht weniger als 95%, 96%, 97%, 98%, 99% oder 99,9% Fructose des sucrosehaltigen Getränks enthält.

4. Verfahren nach einem der vorhergehenden Ansprüche, wobei das Behandeln das Behandeln mit einer SEQ ID NO:1 umfassenden Glucosyltransferase umfasst.

5. Verfahren nach einem der vorhergehenden Ansprüche, wobei das Behandeln das Behandeln mit einem Enzym umfasst, das mindestens 85%, 90%, 95%, 98%, 99% oder 99,9% Sequenz Identität mit der Glucosyltransferase aufweist, die die in SEQ ID NO:1 aufgeführte Aminosequenz aufweist.

6. Verfahren nach einem der vorhergehenden Ansprüche, wobei das Behandeln das Behandeln mit einer immobilisierten Glucosyltransferase umfasst.

7. Verfahren nach einem der vorhergehenden Ansprüche, wobei das Behandeln das Behandeln sich mit einer immobilisierten Glucosyltransferase von *Streptococcus salivarius* umfasst.

8. Verfahren nach einem der vorhergehenden Ansprüche, wobei das Getränk einen Obstsaft umfasst.

9. Verfahren nach einem der vorhergehenden Ansprüche, wobei das Getränk Apfelsaft oder Orangensaft umfasst.

10. Verfahren nach einem der vorhergehenden Ansprüche, wobei das Getränk ein um mindestens 40%, mindestens 50%, mindestens 60%, mindestens 70%, mindestens 80%, mindestens 85% oder mindestens 90% reduziertes Sucroseniveau im Vergleich mit einem Kontrollgetränk, dem die Behandlung fehlt, umfasst.

11. Verfahren nach einem der vorhergehenden Ansprüche, wobei das Getränk mindestens 2, mindestens 2,5, mindestens 3, mindestens 4 oder mindestens 4,3 Gramm Alpha-(1-3)-glucane pro Liter umfasst.

12. Verfahren nach Anspruch 11, wobei das Getränk Orangensaft oder Apfelsaft ist und wobei es nicht weniger als 95%, 96%, 97%, 98%, 99% oder 99,9% Fructose von durchschnittlichem Orangensaft oder Apfelsaft enthält.

## Revendications

1. Procédé de fabrication d'une boisson pauvre en calories, à teneur élevée en fibres insolubles comprenant: le traitement d'une boisson contenant du saccharose avec une glucosyltransférase pour convertir le saccharose en alpha (1-3) glucane pour fabriquer la boisson pauvre en calories, à teneur élevée en fibres insolubles;
le traitement comprenant le traitement avec une enzyme ayant au moins 80% d'identité de séquence par rapport à la glucosyltransférase ayant la séquence d'acides aminés présentée dans SEQ ID n°: 1 et la boisson comprenant une teneur en saccharose réduite d'au moins 30%.

2. Procédé selon la revendication 1, comprenant en outre l'élimination de l'alpha (1-3) glucane pour fabriquer une boisson clarifiée, pauvre en calories.

3. Procédé selon la revendication 1 ou la revendication 2, la boisson pauvre en calories, à teneur élevée en fibres insolubles ne contenant pas moins de 95%, 96%, 97%, 98%, 99%, ou 99,9% de fructose de la boisson contant du saccharose.

4. Procédé selon l'une quelconque des revendications précédentes, le traitement comprenant le traitement avec une glucosyltransférase comprenant SEQ ID n°: 1.

5. Procédé selon l'une quelconque des revendications précédentes, le traitement comprenant le traitement avec une enzyme ayant au moins 85%, 90%, 95%, 98%, 99%, ou 99,9% d'identité de séquence par rapport à la glucosyltransférase ayant la séquence d'acides aminés présentée dans SEQ ID n°: 1.

6. Procédé selon l'une quelconque des revendications précédentes dans lequel le traitement comprend le traitement avec une glucosyltransférase immobilisée.

7. Procédé selon l'une quelconque des revendications précédentes dans lequel le traitement comprend le traitement avec une glucosyltransférase immobilisée provenant de *Streptococcus salivarius.*

8. Procédé selon l'une quelconque des revendications précédentes, dans lequel la boisson comprend un jus de fruit.

9. Procédé selon l'une quelconque des revendications précédentes, dans lequel la boisson comprend du jus de pomme ou du jus d'orange.

10. Procédé selon l'une quelconque des revendications précédentes, dans lequel la boisson comprend une teneur réduite en saccharose, d'au moins 40%, d'au moins 50%, d'au moins 60%, d'au moins 70%, d'au moins 80%, d'au moins 85%, ou d'au moins 90% comparée à une boisson témoin n'ayant pas été traitée.

11. Procédé selon l'une quelconque des revendications précédentes, dans lequel la boisson comprend au moins 2, au moins 2,5, au moins 3, au moins 4, ou au moins 4,3 grammes d'alpha (1-3) glucanes par litre.

12. Procédé selon la revendication 11, dans lequel la boisson est un jus d'orange ou un jus de pomme, et dans lequel elle ne contient pas moins de 95%, 96%, 97%, 98%, 99%, ou 99,9% de fructose du jus d'orange ou du jus de pomme moyen.
